# EUROPÄISCHE PATENTSCHRIFT

(11) **EP 0 756 859 B1**
(45) Veröffentlichungstag und Bekanntmachung des Hinweises auf die Patenterteilung: **16.07.1997**
(21) Anmeldenummer: 96111182.0
(22) Anmeldetag: 11.07.1996
(51) Int. Cl.: A61K 7/06

(54) **Haarbehandlungsmittel**
Hair care composition
Composition pour le soin des cheveux

(30) Priorität: 01.08.1995 DE 19528124
(43) Veröffentlichungstag der Anmeldung: 05.02.1997
(73) Patentinhaber: GOLDWELL GmbH, D-64297 Darmstadt (DE)
(72) Erfinder: Bünning, Einhard, 64342 Seeheim (DE); Pesch, Ferdinand, Dr., 64625 Bensheim (DE)

(56) Entgegenhaltungen:
- EP-A- 0 640 643
- DE-C- 4 426 794
- US-A- 5 180 580

## Beschreibung

Die vorliegende Erfindung betrifft ein Haarbehandlungsmittel, insbesondere ein Haarspray, mit konditionierenden Eigenschaften, das dem Haar insbesondere eine verbesserte Kämmbarkeit verleiht sowie einen angenehmen Griff und ein natürliches Aussehen bewirkt.

Haarkonditionierende Mittel, die die verschiedensten Wirkstoffe enthalten, sind seit langem bekannt und werden in der Praxis intensiv genutzt. Bevorzugte Wirkstoffe sind dabei insbesondere Polymere, vor allem anionische und/oder kationische Polymere, die auf das Haar aufziehen und dort einen konditionierenden Effekt ausüben.

Eine wesentliche Verbesserung der Wirksamkeit solcher haarkonditionierender Mittel wird durch die in der EP-A 640 643 beschriebenen Organopolysiloxane, insbesondere quaternierte Aminoalkyldimethylpolysiloxan/Polyethyloxazolin-Copolymere, erreicht. Hierdurch wird insbesondere die Elastizität des Haares signifikant verbessert.

Es wurde nun gefunden, daß die Wirkung dieser Haarbehandlungsmittel noch weiter verbessert werden kann, wenn man ihnen mindestens zwei weitere Polymere mit unterschiedlicher Ionogenität, ausgewählt aus der Gruppe wasser- und/oder alkohollösliche nichtionische Polymerisate, insbesondere Vinylpyrrolidon-Polymere, weitere kationische, anionische und/oder amphotere (zwitterionische) Polymere, zusetzt.

Dadurch werden vor allem die für den Verbraucher wahrnehmbaren Wirkungen wie Kärmbarkeit, Griff und natürlicher Glanz des Haares nach kurzen Einwirkzeiten wesentlich verstärkt.

Die Erfindung wird in den Patentansprüchen definiert.

Geeignete quaternierte Aminoalkyl-, insbesondere Aminopropyldimethylpolysiloxan/Polyethyloxazolin-Copolymerisate sind solche der Formel 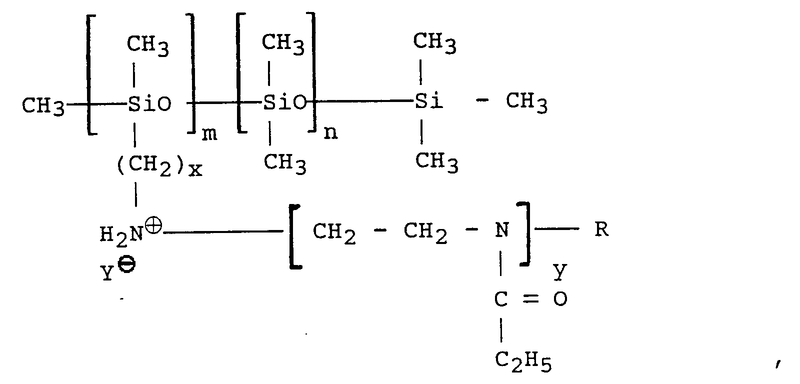 worin m und n jeweils ganze Zahlen von 20 bis 10 000, insbesondere 50 bis 7000, vor allem 100 bis 5000, x eine Zahl zwischen 1 und 5, vorzugsweise 3, und y eine Zahl von 5 bis 30 bedeuten, R eine C₁-C₁₂-Alkyl- oder Arylgruppe, insbesondere eine Methyl-, Ethyl- oder Benzylgruppe, und Y⁻ ein Anion darstellen.

Die Herstellung der erfindungsgemäß zum Einsatz gelangenden Aminoalkyldimethylpolysiloxan/Polyethyloxazolin-Pfropfcopolymerisate ist in der bereits erwähnten EP-A 640 643 beschrieben und erfolgt entsprechend dem folgenden Schema:

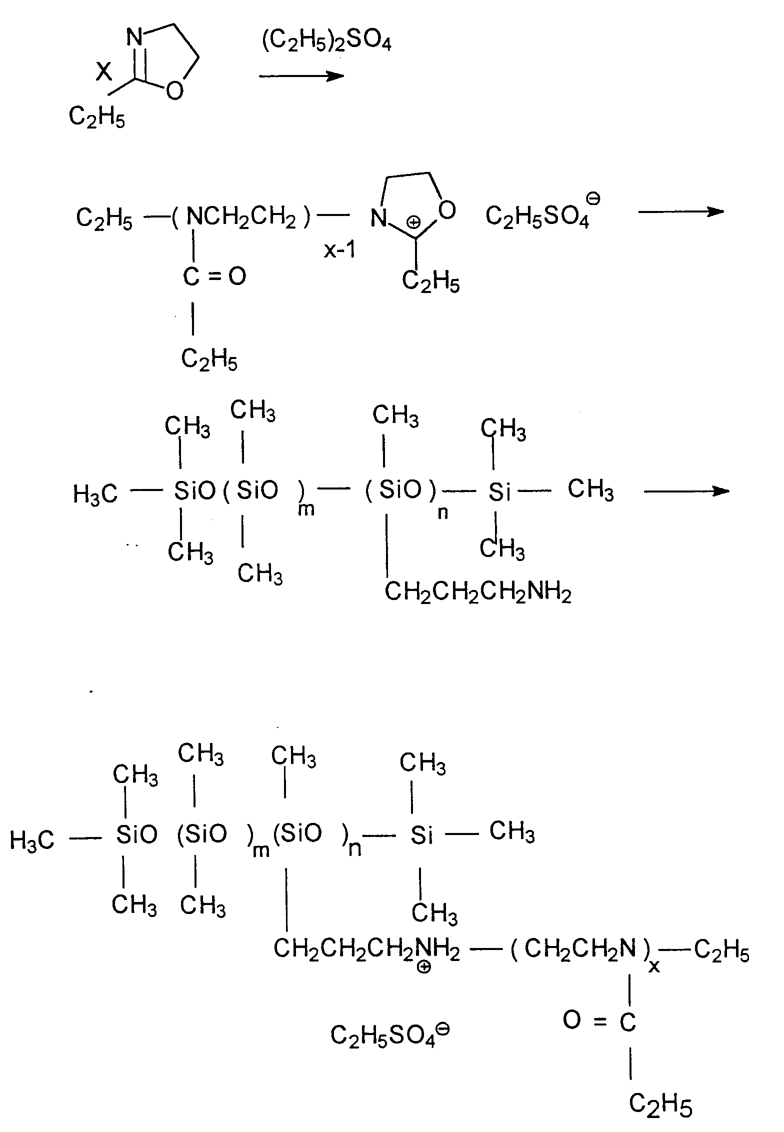

Das Anion Y ⁻der allgemeinen Formel kann selbstverständlich auch ein anderes als das Ethylsulfat-Anion des obengenannten Beispiels sein, d.h. die Quaternierung kann auch mit Methylchlorid, Dimethylsulfat, Benzylchlorid, Dodecylbromid etc. erfolgen.

Ein besonders bevorzugtes Pfropfcopolymerisat der dargestellten Art weist ein Gesamtmolekulargewicht von etwa 50 000 bis etwa 500 000, vorzugsweise etwa 80 000 bis etwa 300 000, insbesondere etwa 100 000 Dalton auf, wobei das Molgewicht des Oxazolin-Segments etwa 2 500 bis etwa 7 500, vorzugsweise etwa 4 000 bis etwa 6 000, insbesondere etwa 5 000 Dalton/Segment beträgt, d.h. der Molanteil bei 20 Einheiten/Molekül liegt. Der bevorzugte Si-Gehalt beträgt, entsprechend der Elementaranalyse, etwa 50 %.

Besonders geeignet sind die unter den Bezeichnungen A-1, A-2 und A-3 auf den Seiten 12 bis 13 der EP-A 640 643 beschriebenen Organopolysiloxane. Der Anteil der Pfropfcopolymerisate in den erfindungsgemäßen Haarbehandlungsmitteln liegt bei etwa 0,1 bis 5, vorzugsweise 0,25 bis 2,5, insbesondere 0,5 bis 1 Gew.-% der Zusammensetzung.

Die erfindungsgemäßen Zusammensetzungen können vorzugsweise noch mindestens ein flüchtiges Silicon enthalten. Flüchtige Silicone sind insbesondere Dimethicone, Phenyl Dimethicone und Cyclomethicone der allgemeinen Formeln: 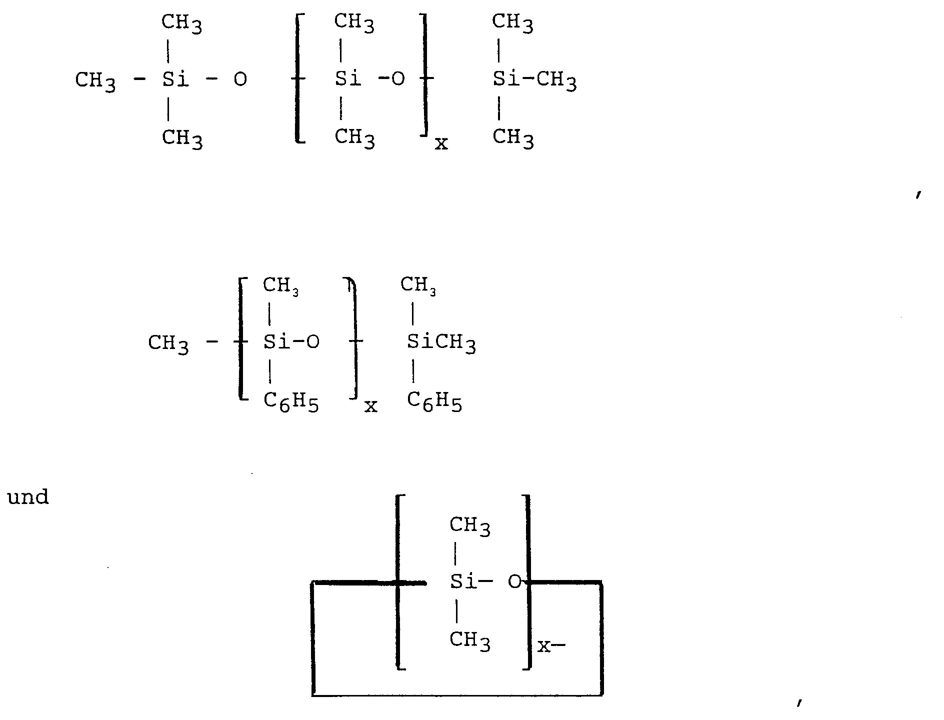 wobei x niedere Zahlen von etwa 1 bis etwa 10, im Falle des erstgenannten Dimethicones auch 0 bis 10, bedeuten.

Solche Produkte sind beispielsweise unter den Bezeichnungen "Dow Corning® 200, 225, 244, 235, 344, 345 Fluid" oder "SF-1202 oder SF-1204 Silicone Fluid" im Handel.

Die Viskositäten dieser flüchtigen Silicone liegen zwischen etwa 0,5 und etwa 500 cSt (0,5-500)·10⁻⁶m²/s bei 25 °C, insbesondere etwa 1 und etwa 100 (1-100)·10⁻⁶m²/s, vorzugsweise etwa 5 bis etwa 50 cSt (5-50)·10⁻⁶m²/s bei 25 °C.

Der erfindungsgemäß anwesende zweite und dritte essentielle Bestandteil kann einmal ein nichtionisches Polymer, vorzugsweise ein alkohol- und/oder wasserlösliches Vinylpyrrolidon-Polymer wie ein Vinylpyrrolidon-Homopolymer oder ein -Copolymerisat, insbesondere mit Vinylacetat, sein, vorausgesetzt, daß das Kriterium der Wasser- bzw. Alkohollöslichkeit erhalten bleibt.
Unter Alkohollöslichkeit ist dabei selbstverständlich die Löslichkeit in niederen Alkoholen wie Ethanol, Propanol-1 oder Propanol-2 zu verstehen.

Geeignete Vinylpyrrolidon-Polymere sind z.B. die unter dem Handelsnamen "Luviskol" bekannten Produkte, beispielsweise die Homopolymerisate "Luviskol K 30, K 60 und K 90" sowie die wasser- bzw. alkohollöslichen Copolymerisate aus Vinylpyrrolidon und Vinylacetat, die unter dem Handelsnamen "Luviskol VA 55 bzw. VA 64" von der BASF AG vertrieben werden.

Weitere geeignete nichtionische Polymere sind Vinylpyrrolidon/Vinylacetat/Vinylpropionat-Copolymere wie "Luviskol VAP 343®", Vinylpyrrolidon/(Meth)Acrylsäureester-Copolymere sowie Chitosan-Derivate.

Ihr Anteil in den erfindungsgemäßen Haarbehandlungsmitteln liegt zwischen etwa 0,25 und etwa 10,0, vorzugsweise 0,5 und 7,5, insbesondere etwa 1 bis 2,5 und 5 Gew.-%, bezogen auf die Gesamtzusammensetzung des Mittels.

Eine große Variationsbreite besteht auch bei dem dritten essentiellen erfindungsgemäßen Bestandteil, dem kationischen und/oder anionischen und/oder amphoteren Polymeren, das in einer Menge zwischen etwa 0,1 und etwa 7,5 bis 10 Gew.-%, vorzugsweise 0,25 bis 5, insbesondere etwa 0,50 bis 7,5 Gew.-%, je nach Charakter des Polymeren, enthalten ist:

Kationische Polymere sind neben den altbekannten quaternären Cellulosederivaten des Typs "Polymer JR" insbesondere quaternisierte Homo- und Copolymere des Dimethyldiallylammoniumchlorids, wie sie unter dem Handelsnamen "Merquat" im Handel sind, quaternäre Vinylpyrrolidon-Copolymere, insbesondere mit Dialkylaminoalkyl(meth)acrylaten, wie sie unter dem Namen "Gafquat" bekannt sind, Copolymerisate aus Vinylpyrrolidon und Vinylimidazoliniummethochlorid, die unter dem Handelsnamen "Luviquat" angeboten werden, Polyamino-Polyamid-Derivate, beispielsweise Copolymere von Adipinsäure-Dimethylaminohydroxypropyldiethylentriamin, wie sie unter dem Namen "Cartaretine F" vertrieben werden, sowie auch bisquaternäre langkettige Ammoniumverbindungen der in der US-PS 4 157 388 beschriebenen Harnstoff-Struktur, die unter dem Handelsnamen "Mirapol A 15" im Handel sind.

Verwiesen wird in diesem Zusammenhang auch auf die in den DE-OSen 25 21 960, 28 11 010, 30 44 738 und 32 17 059 genannten kationaktiven Polymeren sowie die in der EP-A 337 354 auf den Seiten 3 bis 7 beschriebenen Produkte. Es können auch Mischungen verschiedener kationischer Polymerer eingesetzt werden. Der bevorzugte Anteil eines solchen kationischen Polymeren liegt bei 0,1 bis 5, vorzugsweise 0,25 bis 2,5, insbesondere 0,5 bis 1,5 Gew.-% des Mittels.

Als amphotere Polymere, die im Gemisch mit mindestens einem weiteren kationischen, nichtionischen oder anionischen Polymeren zum Einsatz gelangen, seien insbesondere Copolymerisate aus N-Octylacrylamid, (Meth)Acrylsäure und tert.-Butylaminoethylmethacrylat vom Typ "Amphomer®"; Copolymerisate aus Methacryloylethylbetain und Alkylmethacrylaten vom Typ "Yukaformer®", z. B. das Butylmethacrylat-Copolymere "Yukaformer® Am75"; Copolymerisate aus Carboxylgruppen und Sulfongruppen enthaltenden Monomeren, z. B. (Meth)Acrylsäure und Itaconsäure, mit basische Gruppen, insbesondere Aminogruppen enthaltenden Monomeren wie Mono- bzw. Dialkylaminoalkyl(meth)acrylaten bzw. Mono- bzw. Dialkylaminoalkyl(meth)acrylamiden; Copolymere aus N-Octylacrylamid, Methylmethacrylat, Hydroxypropylmethacrylat, N-tert.-Butylaminoethylmethacrylat und Acrylsäure sowie die aus der US-A 3,927,199 bekannten Copolymeren genannt.

Die Menge entspricht in etwa derjenigen des kationischen Polymers.

Besonders geeignete anionische Polymere, deren Menge bei etwa 0,1 und etwa 10, insbesondere etwa 0,25 bis etwa 5 Gew.-% der Gesamtzusammensetzung des Mittels liegt, sind Vinylalkylether-, insbesondere Methylvinylether/Maleinsäure-Copolymere, die durch Hydrolyse von Vinylether/Maleinsäureanhydrid-Copolymeren entstehen und unter der Handelsbezeichnung "Gantrez AN oder ES" vertrieben werden. Diese Polymeren können auch teilverestert sein, beispielsweise "Gantrez ES 225", der Ethylester eines Ethylvinylethers/Maleinsäure-Copolymers, oder der Butyl- oder Isobutylester desselben.

Weitere geeignete anionische Polymere sind insbesondere Vinylacetat/Crotonsäure-oder Vinylacetat/Vinylneodecanoat/Crotonsäure-Copolymere des Typs "Resyn"; Natriumacrylat/Vinylalkohol-Copolymere des Typs "Hydagen F", Natriumpolystyrolsulfonat, z.B. "Flexan 130"; Ethylacrylat/Acrylsäure/N-tert.-Butylacrylamid-Copolymere des Typs "Ultrahold"; Vinylpyrrolidon/Vinylacetat/Itaconsäure-Copolymere, Acrylsäure/Acrylamid-Copolymere bzw. Natriumsalze derselben vom Typ "Reten"; etc.

Selbstverständlich können in den erfindungsgemäßen Zusammensetzungen alle in haarkonditionierenden Mitteln üblichen und bekannten Stoffe mitverwendet werden, vorausgesetzt, daß sie mit den essentiellen Wirkstoffen nicht interferieren. Solche Stoffe sind beispielsweise oberflächenaktive Mittel, Verdickungsmittel, Perlglanz gebende Mittel, Farbstoffe, Riechstoffe, Konservierungsmittel, etc.

Die erfindungsgemäßen Mittel können als Lösungen, Dispersionen, bzw. Emulsionen, Gele oder Schaumaerosole konfektioniert sein; sie liegen jedoch vorzugsweise als Haarsprays, die mit einem Treibmittel abgegeben oder als luftbetriebenes Pumpspray appliziert werden, vor.

Im folgenden werden einige Ausführungsbeispiele von Haarpflegemitteln gegeben, die die Anwendung der Erfindung und deren überlegene Eigenschaften illustrieren sollen.

### Beispiel A

### Haarspray

| **A** | **1** | **2** | **3** | **4** |
|---|---|---|---|---|
| Pfropfcopolymerisat¹ | 0,5 | 0,5 | 0,5 | - |
| Vinylpyrrolidon/Vinylacetat-Copolymer | 5,0 | - | 4,5 | 5,0 |
| Polyquaternium-11² | - | 5,0 | 0,5 | 0,5 |
| Parfum | 0,1 | 0,1 | 0,1 | 0,1 |
| n-Pentan | 29,0 | 29,0 | 29,0 | 29,0 |
| Dimethylether | 27,0 | 27,0 | 27,0 | 27,0 |
| Ethanol | @ 100 | @100 | @ 100 | @100 |
| | | | | |
| Halbseitentest: 1, 2, 4 vs. 3 Jeweils 10 Köpfe, beurteilt von Friseuren. | | | | |
| | | | | |
| Elastizität | + | + | + | + |
| Volumen/Körper | + | + | + | + |
| Kämmbarkeit | + | + | ++ | - |
| Natürliches Gefühl | + | + | ++ | - |

| | | | | |
|---|---|---|---|---|
| ¹) Quaternisiertes Aminopropyldimethylpolysiloxan/Polyethyloxazolin-Pfropfcopolymerisat ("Organopolysiloxan A-1" entsprechend Beispiel 1, S.12, der EP-A 640 643). | | | | |
| ²) Quaternäres Copolymerisat aus Vinylpyrrolidon und Dimethylaminoethylmethacrylat. | | | | |

Die verschiedenen Haarsprays, die alle den gleichen Polymergehalt aufwiesen, wurden im Halbseitentest gegen die Formulierung 3 getestet. Dabei zeigte sich, daß bei vergleichbarer Elastizität bzw. vergleichbarem Volumen die Formulierung 3 eine deutlich verbesserte Kämmfähigkeit aufwies. Hinzu kommt, daß bei guten Festigungsmerkmalen ein ausgesprochen natürliches Haargefühl erzeugt wird. Dies zeichnet sich durch einen gepflegten, angenehmen Griff aus und nicht durch eine ansonsten für Haarsprays typische Rauhigkeit.

### Beispiel B

### Pumpspray

| **B** | **1** | **2** | **3** | **4** |
|---|---|---|---|---|
| Pfropfcopolymerisat¹ | 0,5 | 0,5 | 0,5 | - |
| Vinylpyrrolidon/Vinylacetat-Copolymerisat | 5,0 | - | 4,5 | 5,0 |
| Ultrahold 8®² | - | 5,0 | 0,5 | 0,5 |
| AMP | - | 0,5 | 0,05 | 0,05 |
| Parfum | 0,1 | 0,1 | 0,1 | 0,1 |
| Ethanol | @ 100 | @100 | @ 100 | @100 |
| | | | | |
| Halbseitentest: 1, 2, 4 vs. 3 Jeweils 10 Köpfe, beurteilt von Friseuren. | | | | |
| | | | | |
| Elastizität | + | + | + | + |
| Volumen/Körper | + | ++ | + | + |
| Kämmbarkeit | + | - | ++ | - |
| Natürliches Gefühl | + | - | ++ | - |

| | | | | |
|---|---|---|---|---|
| ¹) Quaternisiertes Aminopropyldimethylpolysiloxan/Polyethyloxazolin-Pfropfcopolymerisat ("Organopolysiloxan A-2" entsprechend Beispiel 2, S.12, der EP-A 640 643). | | | | |
| ²) Acrylat/Acrylamid-Copolymer. | | | | |

Die verschiedenen Haarsprays, die alle den gleichen Polymergehalt aufwiesen, wurden im Halbseitengest gegen die Formulierung 3 getestet. Dabei zeigte sich, daß bei vergleichbarer Elastizität bzw. vergleichbarem Volumen die Formulierung 3 eine deutlich verbesserte Kämmfähigkeit aufweist. Hinzu kommt, daß bei guten Festigungsmerkmalen ein ausgesprochen natürliches Haargefühl erzeugt wird. Dieses zeichnet sich durch einen gepflegten, angenehmen Griff und nicht durch eine ansonsten für Haarsprays typische Rauhigkeit aus. Auch die Durch- bzw. die Auskämmbarkeit bei 3 ist deutlich gegenüber den anderen Formulierungen verbessert.

### Beispiel C

### Haarspray

| **C** | **1** | **2** | **3** | **4** |
|---|---|---|---|---|
| Pfropfcopolymerisat¹ | 0,5 | 0,5 | 0,5 | - |
| Vinylpyrrolidon/Vinylacetat-Copolymerisat | 5,0 | - | 4,5 | 5,0 |
| Ultrahold 8® | - | 5,0 | 0,5 | 0,5 |
| AMP | - | 0,5 | 0,05 | 0,05 |
| Parfum | 0,1 | 0,1 | 0,1 | 0,1 |
| n-Pentan | 29,0 | 29,0 | 29,0 | 29,0 |
| Dimethylether | 27,0 | 27,0 | 27,0 | 27,0 |
| Ethanol | @ 100 | @100 | @ 100 | @100 |
| | | | | |
| Halbseitentest: 1, 2, 4 vs. 3 Jeweils 10 Köpfe, beurteilt von Friseuren. | | | | |
| | | | | |
| Elastizität | + | + | + | + |
| Volumen/Körper | + | ++ | + | + |
| Kämmbarkeit | + | - | ++ | - |
| Natürliches Gefühl | + | - | ++ | - |

| | | | | |
|---|---|---|---|---|
| ¹) Quaternisiertes Aminopropyldimethylpolysiloxan/Polyethyloxazolin-Pfropfcopolymerisat ("Organopolysiloxan A-1" entsprechend Beispiel 1, S.12, der EP-A 640 643). | | | | |

Die verschiedenen Haarsprays, die alle den gleichen Polymergehalt aufwiesen, wurden im Halbseitengest gegen die Formulierung 3 getestet. Dabei zeigte sich, daß bei vergleichbarer Elastizität bzw. vergleichbarem Volumen die Formulierung 3 eine deutlich verbesserte Kämmfähigkeit aufweist. Hinzu kommt, daß bei guten Festigungsmerkmalen ein ausgesprochen natürliches Haargefühl erzeugt wird. Dieses zeichnet sich durch einen gepflegten, angenehmen Griff und nicht durch eine ansonsten für Haarsprays typische Rauhigkeit aus. Auch die Durch- bzw. die Auskämmbarkeit bei 3 ist deutlich gegenüber den anderen Formulierungen verbessert.

### Beispiel D

### Haarsprays

| **D** | **1** | **2** | **3** | **4** |
|---|---|---|---|---|
| Pfropfcopolymerisat¹ | 0,5 | 0,5 | 0,5 | - |
| Vinylpyrrolidon/Vinylacetat-Copolymer (Luviskol VA 64®) | 5,0 | - | 4,5 | 5,0 |
| Polyquaternium-16² | - | 5,0 | 0,5 | 0,5 |
| Parfum | 0,1 | 0,1 | 0,1 | 0,1 |
| n-Pentan | 29,0 | 29,0 | 29,0 | 29,0 |
| Dimethylether | 27,0 | 27,0 | 27,0 | 27,0 |
| Ethanol | @ 100 | @100 | @ 100 | @100 |
| | | | | |
| Halbseitentest: 1, 2, 4 vs. 3 Jeweils 10 Köpfe, beurteilt von Friseuren. | | | | |
| | | | | |
| Elastizität | + | + | + | + |
| Volumen/Körper | + | ++ | + | + |
| Kämmbarkeit | + | - | ++ | - |
| Natürliches Gefühl | + | - | ++ | - |

| | | | | |
|---|---|---|---|---|
| ¹) Quaternäres Aminopropyldimethylpolysiloxan/Polyethyloxazolin-Pfropfcopolymerisat ("Organopolysiloxan A-3" entsprechend Beispiel 3, S.12, der EP-A 640 643). | | | | |
| ²) Quaternäres Vinylimidazol/Vinylpyrrolidon-Copolymer. | | | | |

Die verschiedenen Haarsprays, die alle den gleichen Polymergehalt aufwiesen, wurden im Halbseitengest gegen die Formulierung 3 getestet. Dabei zeigte sich, daß bei vergleichbarer Elastizität bzw. vergleichbarem Volumen die Formulierung 3 eine deutlich verbesserte Kämmfähigkeit aufweist. Hinzu kommt, daß bei guten Festigungsmerkmalen ein ausgesprochen natürliches Haargefühl erzeugt wird. Dieses zeichnet sich durch einen gepflegten, angenehmen Griff und nicht durch eine ansonsten für Haarsprays typische Rauhigkeit aus. Auch die Durch- bzw. die Auskämmbarkeit bei 3 ist deutlich gegenüber den anderen Formulierungen verbessert.

### Beispiel E

### Aerosolschaum-Zusammensetzungen

| **E** | **1** | **2** | **3** | **4** |
|---|---|---|---|---|
| Pfropfcopolymerisat¹ | 0,1 | 0,1 | 0,1 | - |
| Vinylpyrrolidon/Vinylacetat-Copolymer (Luviskol VA 64®) | 4,0 | - | 1,0 | 1,1 |
| Polyquaternium-16 | - | 4,0 | 3,0 | 3,0 |
| Laureth-23 | 0,2 | 0,2 | 0,2 | 0,2 |
| PEG-40 hydriertes Ricinusöl | 0,1 | 0,1 | 0,1 | 0,1 |
| Parfum | 0,1 | 0,1 | 0,1 | 0,1 |
| Propan/Butan | 10,0 | 10,0 | 10,0 | 10,0 |
| Ethanol | 10,0 | 10,0 | 10,0 | 10,0 |
| Wasser | @ 100 | @100 | @ 100 | @100 |
| | | | | |
| Halbseitentest: 1, 2, 4 vs. 3 Jeweils 10 Köpfe, beurteilt von Friseuren. | | | | |
| | | | | |
| Elastizität | + | + | + | + |
| Volumen/Körper | + | ++ | + | + |
| Kämmbarkeit | + | - | ++ | - |
| Natürliches Gefühl | + | - | ++ | - |

| | | | | |
|---|---|---|---|---|
| ¹) Quaternisiertes Aminopropyldimethylpolysiloxan/Polyethyloxazolin-Pfropfcopolymerisat ("Organopolysiloxan A-1" entsprechend Beispiel 1, S.12, der EP-A 640 643). | | | | |

Die verschiedenen Schäume, die alle den gleichen Polymergehalt aufwiesen, wurden im Halbseitengest gegen die Formulierung 3 getestet. Dabei zeigte sich, daß bei vergleichbarer Elastizität bzw. vergleichbarem Volumen die Formulierung 3 eine deutlich verbesserte Kämmfähigkeit aufweist. Hinzu kommt, daß bei guten Festigungsmerkmalen ein ausgesprochen natürliches Haargefühl erzeugt wird. Dieses zeichnet sich durch einen gepflegten, angenehmen Griff und nicht durch eine ansonsten für festigende Schäume typische Rauhigkeit aus. Auch die Durch- bzw. die Auskämmbarkeit bei 3 ist deutlich gegenüber den anderen Formulierungen verbessert.

### Beispiel F

### Schaumaerosol-Zusammensetzungen

| **F** | **1** | **2** | **3** | **4** |
|---|---|---|---|---|
| Pfropfcopolymerisat¹ | 0,1 | 0,1 | 0,1 | - |
| Vinylacetat/Crotonsäure-Copolymer (Aristoflex A®) | 4,0 | - | 3,0 | 3,0 |
| NaOH | 1,3 | - | 1,0 | 1,0 |
| Polyquaternium-11² | - | 4,0 | 1,0 | 1,1 |
| Laureth-23 | 0,2 | 0,2 | 0,2 | 0,2 |
| PEG-40 hydriertes Ricinusöl | 0,1 | 0,1 | 0,1 | 0,1 |
| Parfum | 0,1 | 0,1 | 0,1 | 0,1 |
| Propan/Butan | 10,0 | 10,0 | 10,0 | 10,0 |
| Ethanol | 10,0 | 10,0 | 10,0 | 10,0 |
| Wasser | @ 100 | @100 | @ 100 | @100 |
| | | | | |
| Halbseitentest: 1, 2, 4 vs. 3 Jeweils 10 Köpfe, beurteilt von Friseuren. | | | | |
| | | | | |
| Elastizität | + | + | + | + |
| Volumen/Körper | + | ++ | + | + |
| Kämmbarkeit | + | - | ++ | - |
| Natürliches Gefühl | + | - | ++ | - |

| | | | | |
|---|---|---|---|---|
| ¹) Quaternisiertes Aminopropyldimethylpolysiloxan/Polyethyloxazolin-Pfropfcopolymerisat ("Organopolysiloxan A-2" entsprechend Beispiel 2, S.12, der EP-A 640 643). | | | | |
| ²) Quaternäres Copolymerisat aus Vinylpyrrolidon und Dimethylaminoethylmethacrylat. | | | | |

Die verschiedenen Schäume, die alle den gleichen Polymergehalt aufwiesen, wurden im Halbseitengest gegen die Formulierung 3 getestet. Dabei zeigte sich, daß bei vergleichbarer Elastizität bzw. vergleichbarem Volumen die Formulierung 3 eine deutlich verbesserte Kämmfähigkeit aufweist. Hinzu kommt, daß bei guten Festigungsmerkmalen ein ausgesprochen natürliches Haargefühl erzeugt wird. Dieses zeichnet sich durch einen gepflegten, angenehmen Griff und nicht durch eine ansonsten für festigende Schäume typische Rauhigkeit aus. Auch die Durch- bzw. die Auskämmbarkeit bei 3 ist deutlich gegenüber den anderen Formulierungen verbessert.

## Patentansprüche

1. Mittel zur Haarbehandlung, enthaltend in einer wäßrigen, wäßrig-alkoholischen oder alkoholischen Grundlage
a) 0,05 bis 5 Gew.-% mindestens eines quaternierten Aminoalkyldimethylpolysiloxan/Polyethyloxazolin-Copolymerisats der Formel 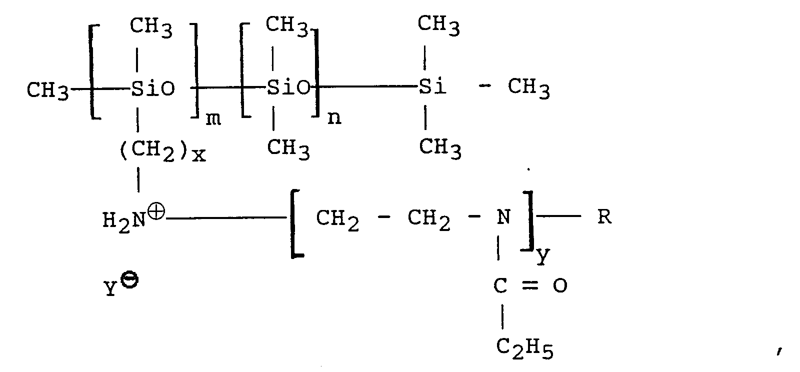 wobei m und n jeweils ganze Zahlen von 20 bis 10 000, x eine Zahl zwischen 1 und 5 und y eine Zahl von 5 bis 500 bedeuten, R eine Alkylgruppe mit 1 bis 12 Kohlenstoffatomen oder einen Arylrest und Y^{⊖} ein Anion darstellen, und
b) mindestens zwei weitere Polymere mit voneinander unterschiedlicher Ionogenität, ausgewählt aus der Gruppe nichtionische, weitere kationische, anionische und/oder amphotere (zwitterionische) Polymere, in einer Menge von 0,1 bis 10 Gew.-%,
jeweils berechnet auf die Gesamtzusammensetzung der Mittel.

2. Mittel nach Anspruch 1, dadurch gekennzeichnet, daß es als Bestandteil b) ein Gemisch aus
(i) 0,25 bis 10 Gew.-% mindestens eines nichtionischen Polymeren, und
(ii) 0,25 bis 5 Gew.-% mindestens eines weiteren kationischen Polymeren,
jeweils berechnet auf die Gesamtzusammensetzung des Mittels, enthält.

3. Mittel nach Anspruch 1, dadurch gekennzeichnet, daß es als Bestandteil b) ein Gemisch aus
(i) 0,25 bis 10 Gew.-% mindestens eines nichtionischen Polymeren, und
(ii) 0,1 bis 10 Gew.-% mindestens eines anionischen Polymeren,
jeweils berechnet auf die Gesamtzusammensetzung des Mittels, enthält.

4. Mittel nach einem oder mehreren der Ansprüche 1 bis 3, dadurch gekennzeichnet, daß es als nichtionisches Polymer ein Vinylpyrrolidon/Vinylacetat-Copolymerisat enthält.

5. Mittel nach Anspruch 1, dadurch gekennzeichnet, daß es als Bestandteil b) ein Gemisch aus
(i) 0,25 bis 5 Gew.-% mindestens eines weiteren kationischen Polymeren, und
(ii) 0,1 bis 10 Gew.-% mindestens eines anionischen Polymeren,
jeweils berechnet auf die Gesamtzusammensetzung des Mittels, enthält.

6. Mittel nach einem oder mehreren der Ansprüche 1 bis 5, dadurch gekennzeichnet, daß es als Lösungsmittel Ethanol, n-Propanol und/oder Isopropylalkohol enthält.

7. Mittel nach einem oder mehreren der Ansprüche 1 bis 6, dadurch gekennzeichnet, daß es zusätzlich ein flüchtiges Silicon enthält.

8. Mittel nach einem oder mehreren der Ansprüche 1 bis 7, dadurch gekennzeichnet, daß es in Form einer Aerosolzusammensetzung vorliegt, deren Wassergehalt weniger als 20 Gew.-%, berechnet auf die Gesamtzusammensetzung des Mittels, beträgt.

9. Mittel nach einem oder mehreren der Ansprüche 1 bis 8, dadurch gekennzeichnet, daß es in Form einer Aerosolschaum-Zusammensetzung auf wäßriger oder wäßrig-alkoholischer Grundlage vorliegt.

## Claims

1. Hair treatment composition, comprising in an aqueous, aqueous-alcoholic or alcoholic medium
a) 0.05% to 5% by wt. of at least one quaternized amino-alkyl dimethyl polysiloxane/polyethyl oxazoline copolymer of the formula 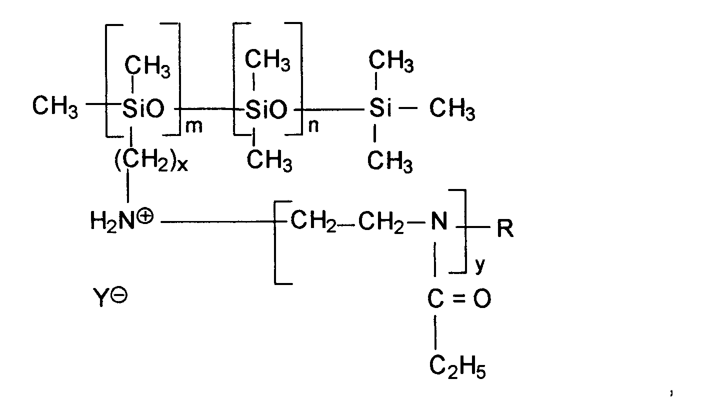 where m and n each denote whole numbers from 20 to 10,000, x is a number from 1 to 5, and y is a number from 5 to 500, R represents an alkyl group bearing 1 to 12 carbon atoms or an aryl residue, and Y⁻ is an anion, and
b) at least two further polymers of different ionogenicity, selected from the group of nonionic, other cationic, anionic and (or) amphoteric (zwitterionic) polymers, in a proportion from 0.1% to 10% by wt.,
each being calculated to the total composition.

2. Composition according to claim 1, comprising as ingredient b) a mixture of
(i) 0.25% to 10% by wt. of at least one nonionic polymer, and
(ii) 0.25% to 5% by wt. of at least one additional cationic polymer,
each calculated to the total composition.

3. Composition according to claim 1, comprising as ingredient b) a mixture of
(i) 0.25% to 10% by wt. of at least one nonionic polymer, and
(ii) 0.1% to 10% by wt. of at least one anionic polymer,
each calculated to the total composition.

4. Composition according to one or more of claims 1 to 3, comprising as nonionic polymer a vinyl pyrrolidone/vinyl acetate copolymer.

5. Composition according to claim 1, comprising as ingredient b) a mixture of
(i) 0.25% to 5% by wt. of at least one additional cationic polymer, and
(ii) 0.1% to 10% by wt. of at least one anionic polymer,
each calculated to the total composition.

6. Composition according to one or more of claims 1 to 5, containing ethanol, n-propanol and (or) isopropyl alcohol.

7. Composition according to one or more of claims 1 to 6, comprising a volatile silicone.

8. Composition according to one or more of the claims 1 to 7, being present as an aerosol composition having a water content of less than 20% by wt., calculated to the total composition.

9. Composition according to one or more of claims 1 to 8, being present as an aerosol foam composition in an aqueous or aqueous-alcoholic medium.

## Revendications

1. Produit pour le traitement des cheveux, contenant, dans une base aqueuse, aqueuse-alcoolique ou alcoolique,
a) de 0,05 à 5 % en poids d'au moins un copolymère aminoalkyldiméthylpolysiloxane/polyéthyloxazoline rendu quaternaire de formule 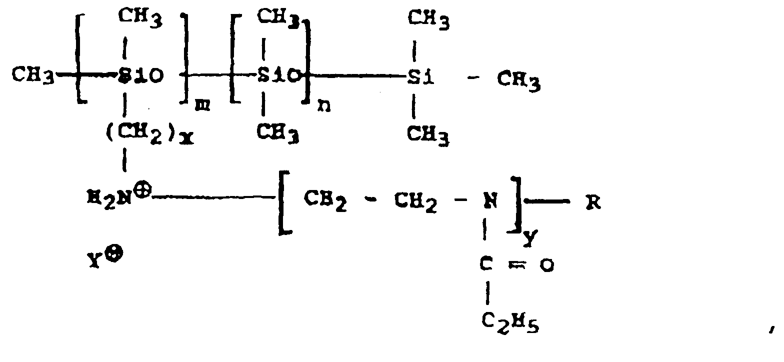 dans laquelle m et n représentent chacun un nombre entier allant de 20 à 10 000, x représente un nombre compris entre 1 et 5, et y représente un nombre allant de 5 à 500, R représente un groupe alkyle ayant de 1 à 12 atomes de carbone ou un radical aryle, et Y⁽⁻⁾ représente un anion, et
b) au moins deux autres polymères à caractères ionogènes différents l'un de l'autre, choisis dans le groupe constitué par des polymères non ioniques, d'autres polymères cationiques, des polymères anioniques et/ou amphotères (dipolaires), en une proportion de 0,1 à 10 % en poids,
dans chaque cas par rapport à la composition totale des produits.

2. Produit selon la revendication 1, caractérisé en ce qu'il contient, en tant que composant b), un mélange de
(I) 0,25 à 10 % en poids d'au moins un polymère non ionique, et
(II) 0,25 à 5 % en poids d'au moins un autre polymère cationique,
dans chaque cas par rapport à la composition totale du produit.

3. Produit selon la revendication 1, caractérisé en ce qu'il contient, en tant que composant b), un mélange de
(I) 0,25 à 10 % en poids d'au moins un polymère non ionique, et
(II) 0,1 à 10 % en poids d'au moins un polymère anionique,
dans chaque cas par rapport à la composition totale du produit.

4. Produit selon une ou plusieurs des revendications 1 à 3, caractérisé en ce qu'il contient, en tant que polymère non ionique, un copolymère vinylpyrrolidone/acétate de vinyle.

5. Produit selon la revendication 1, caractérisé en ce qu'il contient, en tant que composant b), un mélange de
(I) 0,25 à 5 % en poids d'au moins un autre polymère cationique, et
(II) 0,1 à 10 % en poids d'au moins un polymère anionique,
dans chaque cas par rapport à la composition totale du produit.

6. Produit selon une ou plusieurs des revendications 1 à 5, caractérisé en ce qu'il contient en tant que solvant de l'éthanol, du n-propanol et/ou de l'alcool isopropylique.

7. Produit selon une ou plusieurs des revendications 1 à 6, caractérisé en ce qu'il contient en outre une silicone volatile.

8. Produit selon une ou plusieurs des revendications 1 à 7, caractérisé en ce qu'il se présente sous forme d'une composition pour aérosol, dont la teneur en eau est inférieure à 20 % en poids, par rapport à la composition totale du produit.

9. Produit selon une ou plusieurs des revendications 1 à 8, caractérisé en ce qu'il se présente sous forme d'une composition de mousse pour aérosol, à base aqueuse ou aqueuse-alcoolique.
